# EUROPÄISCHE PATENTSCHRIFT

(11) **EP 0 918 744 B1**
(45) Veröffentlichungstag und Bekanntmachung des Hinweises auf die Patenterteilung: **04.08.2004**
(21) Anmeldenummer: 97923022.4
(22) Anmeldetag: 07.05.1997
(51) Int. Cl.: C07C 225/20

(54) **RACEMATTRENNUNG VON KETAMIN**
RACEMIC SEPARATION OF KETAMINE
SEPARATION RACEMIQUE DE KETAMINE

(30) Priorität: 15.05.1996 DE 19619665
(43) Veröffentlichungstag der Anmeldung: 02.06.1999
(73) Patentinhaber: Gödecke GmbH, 76139 Karlsruhe (DE); CU Chemie Uetikon GmbH, 77933 Lahr (DE)
(72) Erfinder: STEINER, Klaus, D-79312 Emmendingen (DE); GANGKOFNER, Stefan, D-77933 Lahr (DE); GRUNENWALD, Jean-Marie, F-67230 Kertzfeld-Benfeld (FR)
(74) Vertreter: Mansmann, Ivo
(86) Internationale Anmeldenummer: PCT/EP1997/002360
(87) Internationale Veröffentlichungsnummer: WO 1997/043244

(56) Entgegenhaltungen:
- DE-A- 2 062 620

## Beschreibung

Die vorliegende Erfindung betrifft ein verbessertes Verfahren zur Spaltung von racemischem 2-(o-Chlorphenyl)-2-methylaminocyclohexanon (Ketamin).

Pharmakologische Untersuchungen zeigen deutliche qualitative und quantitative Unterschiede zwischen dem R- und S-Ketamin Enantiomeren auf. Sowohl präklinisch als auch in einer klinischen Studie schnitt S-Ketamin immer besser ab als der Antipode oder das Racemat. Unter diesen Gesichtspunkten ist die ausschließliche therapeutische Verwendung des Enantiomeren zu bevorzugen und das Racemat zu trennen. Im folgenden ist daher immer das S-Enantiomer gemeint, welches als Salz in der S-(+) - und als reine Base in der S-(-) - Konfiguration vorliegt.

Aus der deutschen Offenlegungsschrift DE-A-2 062 620 ist ein Verfahren zur Spaltung von racemischem 2-(o-Chlorphenyl)-2-methylaminocyclohexanon bekannt, bei dem racemisches 2-(o-Chlorphenyl)-2-methylaminocyclohexanon unter Verwendung einer enantiomeren Form von Weinsäure in einem Lösungsmittelgemisch aus Wasser und Aceton umgesetzt, das gebildete Weinsäuresalz durch Filtration und anschließendes zweimaliges Umkristallisieren mit Acetonitril isoliert wird, worauf das eine Isomere aus dem Weinsäuresalz durch Umsetzen mit Alkali freigesetzt wird. Das Verfahren krankt jedoch an schlechten Ausbeuten, an der Verwendung von giftigen Lösungsmitteln, an unreinen Produkten und an der Notwendigkeit der Durchführung vieler Verfahrensschritte.

Aufgabe der vorliegenden Erfindung war es deshalb, ein verbessertes Verfahren zur Spaltung von racemischem 2-(o-Chlorphenyl)-2-methylaminocyclohexanon anzugeben, bei dem die obigen Schwierigkeiten nicht auftreten.

Gegenstand der vorliegenden Erfindung ist folglich ein Verfahren zur Spaltung von racemischem 2-(o-Chlorphenyl) -2-methylaminocyclohexanon der Formel: 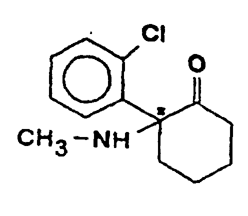 worin * ein asymmetrisches Kohlenstoffatom bedeutet, das die folgenden Stufen umfaßt:
1. Umsetzen von racemischem 2-(o-Chlorphenyl)-2-methylamino-cyclohexanon mit einer enantiomeren Form von Weinsäure,
2. Isolieren des gebildeten Weinsäuresalzes von 2-(o-Chlorphenyl)-2=methylaminocyclohexanon und
3. Umsetzen des isolierten Weinsäuresalzes von 2-(o-Chlorphenyl)-2-methylaminocyclohexanon mit Alkali, wobei ein Isomeres des 2-(o-Chlorphenyl)-2-methylaminocyclohexanons isoliert werden kann,
wobei die Stufen 1 und 2 in Wasser oder einem Gemisch aus Wasser und Isopropanol durchgeführt werden.

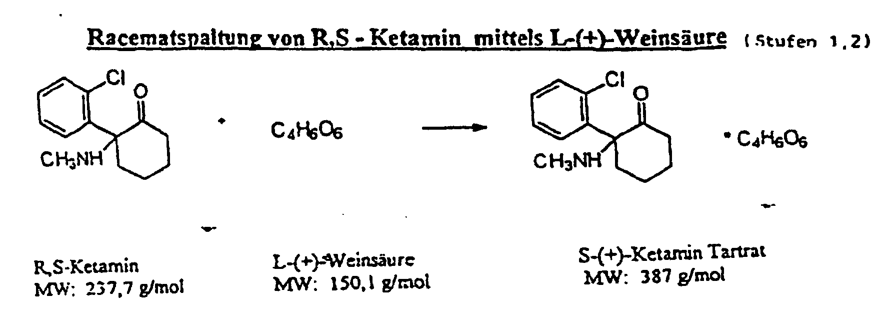

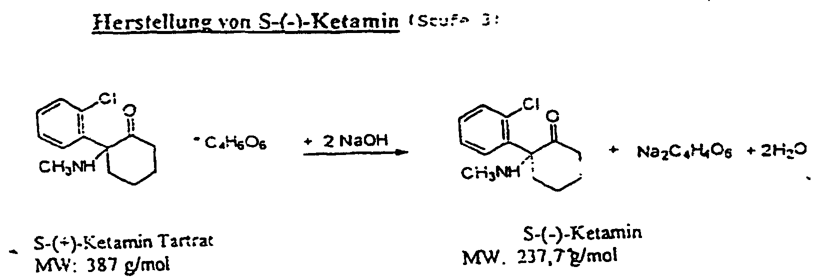

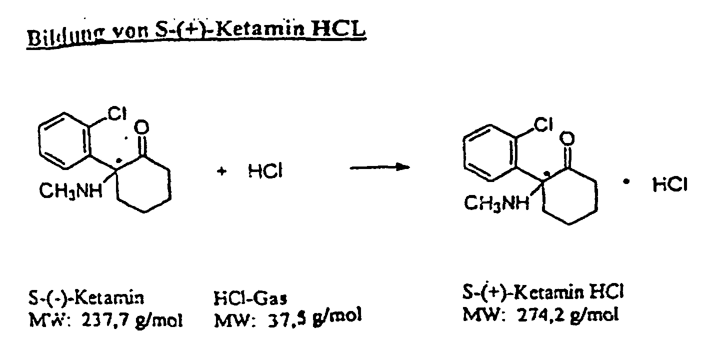

Im Rahmen des erfindungsgemäßen Verfahrens wird racemisches 2-(o-Chlorphenyl)-2-methylaminocyclohexanon in einer ersten Stufe mit einer enantiomeren Form von Weinsäure unter Bildung eines Weinsäuresalzes von 2-(o-Chlorphenyl)-2-methylaminocyclohexanon umgesetzt. Als Lösungsmittel in dieser Reaktion wird Wasser oder ein Gemisch von Wasser und Isopropanol verwendet. Das Verhältnis von Wasser und Isopropanol beträgt vorzugsweise 1,5 : 1.

In einer zweiten Stufe wird das gebildete Weinsäuresalz von 2-(o-Chlorphenyl)-2-methylaminocyclohexanon isoliert. Dies erfolgt vorzugsweise durch Filtration. Auf ein Umkristallisieren des isolierten Weinsäuresalzes von 2-(o-Chlorphenyl)-2-methylaminocyclohexanon kann verzichtet werden.

In der dritten Stufe wird das in Stufe 2 erhaltene Tartrat des 2-(o-Chlorphenyl)-2-methylaminocyclohexanons mit Alkali umgesetzt, wobei ein kristallines Produkt erhalten wird.
Hierbei handelt es sich um isomerenreines 2-(o-Chlorphenyl)-2-methylaminocyclohexanon. Dieses wird beispielsweise durch Filtration gewonnen.

Anschließend kann das isomerenreine Ketamin mit HCl in das entsprechende Hydrochlorid überführt werden.

Im Folgenden wird die vorliegende Erfindung anhand von Beispielen weiter veranschaulicht. Es ist selbstverständlich, daß die Beispiele die vorliegende Erfindung in keiner Weise einschränken.

### Beispiel 1

Es werden 125 ml Wasser vorgelegt und anschließend 31,5 g (0,21 mol) L-(+)-Weinsäure und 50 g (0,21 mol) R,S-Ketamin zugegeben.
Diese Mischung wird unter Rühren auf 50-60°C erwärmt, bis eine klare Lösung entsteht.
Nach Abkühlen auf Raumtemperatur unter Rühren und Nachrühren Über Nacht werden die gebildeten Kristalle abgesaugt. Anschließend wird das Kristallisat erst mit 8. ml Wasser (1-6°C) und anschließend zweimal mit jeweils 20 ml Aceton nachgewaschen.
Ein Trocknen im Umlufttrockenschrank (erst bei RT, später bei 50-60°C) liefert 31,79 g Tartrat (78,23% d.Th).

### Beispiel 2

Es werden 150 ml Wasser vorgelegt und anschließend mit 39,8 g (0,27 mol) L-(+)-Weinsäure und 50 g (0,21 mol) R,S-Ketamin versetzt.
Diese Mischung wird unter Rühren auf 50-60°C erwärmt, bis eine klare Lösung entsteht.
Nach Abkühlen auf Raumtemperatur unter Rühren und Nachrühren über Nacht werden die gebildeten Kristalle abgesaugt. Anschließend wird das Kristallisat nacheinander mit 8 ml Wasser (1-6°C) und anschließend zweimal mit jeweils 20 ml Aceton nachgewaschen.
Ein Trocknen im Umlufttrockenschrank (erst bei RT, später bei 50-60°C) liefert 32,58 g Tartrat (80,02% d. Th).

### Beispiel 3

Es werden 150 ml Wasser und 50 ml Isopropanol vorgelegt. Nach Zugabe von 39,8 g (0,27 mol) L-(+)-Weinsäure und 50 g (0,21 mol) R,S-Ketamin wird die Mischung unter Rühren unter Rückfluß so weit erwärmt, bis eine Lösung entsteht (ggf. Wasser zufügen, bis alles gelöst ist).
Anschließend wird die Lösung unter Rühren auf Raumtemperatur abkühlen gelassen und über Nacht nachgerührt. Die abgesaugten Kristalle werden mit einem 1:2 Gemisch aus 20 ml Wasser/Isopropanol nachgewaschen und im Umlufttrockenschrank getrocknet (erst bei RT, später bei 50-60°C). Man erhält 24,45 g Tartrat (62,63% d. Th.).

### Beispiel 4

Man legt 75 ml Wasser und 50 ml Isopropanol vor und gibt anschließend 39,8 g (0,27 mol) L-(+)-Weinsäure zu. Die Mischung wird unter Rühren auf Rückflußtemperatur erwärmt, bis eine klare Lösung entsteht.
Nach Abkühlen auf Raumtemperatur unter Rühren und Nachrühren über Nacht werden die gebildeten Kristalle abgesaugt. Anschließend wird das Kristallisat mit einem 1:2 Gemisch aus 20 ml Wasser/Isopropanol nachgewaschen. Nach Trocknen im Umlufttrockenschrank (erst bei RT, später bei 50-60°C) erhält man 34,84 g Tartrat (85,74% d. Th.) .

### Beispiel 5

20 g eines in Beispiel 3 erhaltenen S-(+)-Tartrats werden in 100 ml Wasser bei 30-40°C gelöst. Mit ca. 7 ml 50%-iger NaOH wird eine S-(-)-Ketamin Base bis ca. pH 13 ausgefällt. Es wird abgesaugt und mit Wasser bis pH 7-8 neutral gewaschen. Anschließend wird ca. 24 h bei 50°C im Umlufttrockenschrank getrocknet. Man erhält 11,93 g S-(-)-Ketamin (97,79% d. Th.).

### Beispiel 6

5 g des in Beispiel 5 erhaltenen S-(-)-Ketamins werden in 50 ml Isopropanol bei ca. 50°C gelöst und ggf. über Kieselgur abgesaugt. Anschließend wird bei 50-60°C HCl-Gas eingeleitet, bis ein pH-Wert von 0-1 erreicht ist. Man läßt auf Raumtemperatur abkühlen, saugt ab und wäscht mit ca. 5 ml Isopropanol. Das Feuchtprodukt wird über Nacht bei ca. 50°C im Umlufttrockenschrank getrocknet. Man erhalt 5,09 g S-(+) Ketamin HCl (88,06% d. Th.).

## Patentansprüche

1. Verfahren zur Spaltung vom racemischem 2-(o-Chlorphenyl)-2-methylaminocyclohexanon der Formel: 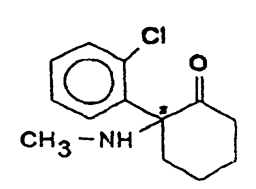 worin * ein asymmetrisches Kohlenstoffatom bedeutet,
wobei man das Salz von racemischem 2-(o-Chlorphenyl)-2-methylaminocyclohexanon mit einer enantiomeren Form der Weinsäure aus einem Lösungsmittel kristallisiert und anschließend das isolierte Salz mit Alkali umsetzt,
**dadurch gekennzeichnet,**
**dass** das Lösungsmittel Wasser oder ein Gemisch von Wasser und Isopropanol ist.

2. Verfahren nach Anspruch 1, wobei als Lösungsmittel ein Gemisch von Wasser und Isopropanol im Volumenverhältnis von 1,5 : 1 verwendet wird.

3. Verfahren nach Anspruch 1 oder 2, wobei als Alkali Natronlauge verwendet wird.

4. Verfahren nach Anspruch 1 bis 3, wobei das erhaltene freie Isomere von 2-(o-Chlorphenyl)-2-methylamino-cyclohexanon anschließend durch Umsetzung mit Chlorwasserstoffsäure in das Hydrochloridsalz des jeweiligen Isomeren übergeführt wird.

## Claims

1. Method for the separation of racemic 2-(o-chlorophenyl) 2-methylamino cyclohexanone with the formula: 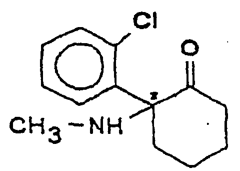 where * denotes an asymmetric carbon atom, in which the racemic 2-(o-chlorophenyl) 2-methylamino cyclohexanone salt is crystallised with an enantiomeric form of tartaric acid from a solvent, after which the separated salt is reacted with alkali,
**characterised in that** the solvent is water or a mixture of water and isopropanol.

2. Method in accordance with Claim 1, where a mixture of water and isopropanol in a volume ratio of 1.5 : 1 is used as the solvent.

3. Method in accordance with Claim 1 or 2, where sodium hydroxide solution is used as the alkali.

4. Method in accordance with Claims 1 to 3, where the free isomer of 2-(o-chlorophenyl) 2-methylamino cyclohexanone is subsequently transformed into the hydrochloride of the corresponding isomer by reacting it with hydrochloric acid.

## Revendications

1. Procédé pour cliver la 2-(o-chlorophényl)-2-méthylaminocyclohexanone racémique de formule : 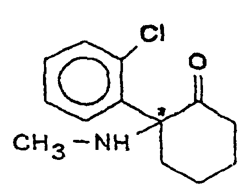 où * signifie un atome de carbone asymétrique, dans lequel on fait cristalliser le sel de 2-(o-chlorophényl)-2-méthylaminocyclohexanone racémique avec une forme énantiomère de l'acide tartrique dans un solvant puis on fait réagir le sel isolé avec un alcali,
**caractérisé en ce que** le solvant est l'eau ou un mélange d'eau et d'isopropanol.

2. Procédé selon la revendication 1, dans lequel un mélange d'eau et d'isopropanol est employé comme solvant dans un rapport volumique de 1,5:1.

3. Procédé selon la revendication 1 ou 2, dans lequel la soude caustique est employé comme alcali.

4. Procédé selon la revendication 1 à 3, dans lequel l'isomère libre obtenu de 2-(o-chlorophényl)-2-méthylaminocyclohexanone est transformé ensuite en le sel chlorhydrate de l'isomère en question par réaction avec l'acide chlorhydrique.
